(19) Europäisches Patentamt
European Patent Office
Office européen des brevets

(11) Veröffentlichungsnummer: **0 062 199**
**B1**

# (12) EUROPÄISCHE PATENTSCHRIFT

(45) Veröffentlichungstag der Patentschrift: **20.06.84**

(51) Int. Cl.³: **C 07 D 471/08,** A 61 K 31/445 // (C07D471/08, 221/00, 221/00)

(21) Anmeldenummer: **82102271.2**

(22) Anmeldetag: **19.03.82**

(54) **N-(4-Aminobenzoyl)-N'-benzylbispidin, seine Herstellung und dieses enthaltende Arzneimittel.**

(30) Priorität: **27.03.81 DE 3112055**

(43) Veröffentlichungstag der Anmeldung: **13.10.82 Patentblatt 82/41**

(45) Bekanntmachung des Hinweises auf die Patenterteilung: **20.06.84 Patentblatt 84/25**

(84) Benannte Vertragsstaaten: **AT BE CH DE FR GB IT LI LU NL SE**

(56) Entgegenhaltungen:
**DE - A - 2 428 792**
**DE - A - 2 726 571**

(73) Patentinhaber: **BASF Aktiengesellschaft, Carl-Bosch-Strasse 38, D-6700 Ludwigshafen (DE)**

(72) Erfinder: **Binnig, Fritz, Dr., Raiffeisenstrasse 31, D-6701 Fussgoenheim (DE)**
Erfinder: **Mueller, Claus D., Dr., Odenwaldring 84, D-6806 Viernheim (DE)**
Erfinder: **Raschack, Manfred, Dr., Donnersbergstrasse 7, D-6714 Weisenheim am Sand (DE)**
Erfinder: **Von Philipsborn, Gerda, Dr., Naechstenbacher Weg 35, D-6940 Weinheim (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**EP 0 062 199 B1**

ACTORUM AG

**Beschreibung**

Die Erfindung betrifft ein neues Bispidinderivat, dessen Herstellung sowie Arzneimittel, die diese neue Substanz enthalten.

Es ist bereits bekannt, dass eine Reihe von Bispidinderivaten antiarrhythmische Eigenschaften besitzen [vgl. DE-OS 2 428 792, DE-OS 2 726 571, J. Med. Chem. 20, 1668 (1977)].

Es wurde nun ein neues Bispidinderivat gefunden, das die bekannten Derivate in ihrer Wirkung übertrifft.

Gegenstand der Erfindung sind N-(4-Aminobenzoyl)-N'-benzylbispidin der Formel I

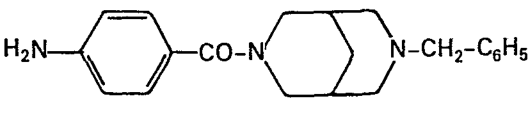 (I),

sowie dessen Salze mit physiologisch verträglichen Säuren.

Als physiologisch verträgliche Säuren kommen z.B. in Frage: Salzsäure, Schwefelsäure, Phosphorsäure, Essigsäure, Malonsäure, Bernsteinsäure, Fumarsäure, Maleinsäure, Citronensäure, Weinsäure, Milchsäure und Diamidosulfonsäure.

Gegenstand der Erfindung ist weiter ein Verfahren zur Herstellung des Bispidinderivats der Formel I und dessen Salzen mit physiologisch verträglichen Säuren, welches darin besteht, dass man N-Monobenzylbispidin mit einer Verbindung der Formel II

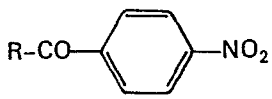 (II),

worin R ein Halogenatom bedeutet, umsetzt, die Nitrogruppe reduziert und die so erhaltene Verbindung gegebenenfalls in ihre Salze mit physiologisch verträglichen Säuren überführt.

Die Umsetzung des N-Monobenzylbispidins mit den Verbindungen II kann beispielsweise mit Natriumhydrid in Dimethylformamid; Natriumhydroxid in Wasser oder Ethanol; Natriumcarbonat in Butanol oder Amylalkohol; Kaliumcarbonat in Wasser, Methanol, Ethanol, Isopropanol, Butanol, Amylalkohol, Aceton, Acetonitril, Toluol, Dimethylformamid, Dimethylsulfoxid oder Tetrahydrofuran; Natriummethylat in Methanol; Natriumisopropylat in Isopropanol; Kalium-tert.-butylat in tert. Butanol, Tetrahydrofuran oder Dimethylsulfoxid; Natriumamid in Toluol oder Xylol erfolgen. Am besten gelingt die Reaktion mit Natriumhydrid in Dimethylformamid. Sie wird in der Regel bei Raumtemperatur durchgeführt.

Die Reduktion der Nitrogruppe erfolgt am besten katalytisch mit Wasserstoff. Als Katalysator eignet sich insbesondere Platin.

Als Lösungsmittel kommen niedere Alkohole, insbesondere Ethanol, in Betracht.

Schliesslich betrifft die Erfindung Arzneimittel, welche das Bispidinderivat der Formel I oder dessen Salze mit physiologisch verträglichen Säuren enthalten.

Die neuen Verbindungen können in üblicher Weise oral oder parenteral (intravenös, intramuskulär, intraperitoneal) verabfolgt werden.

Die Dosierung hängt vom Alter, Zustand und Gewicht des Patienten sowie von der Applikationsart ab. In der Regel beträgt die tägliche Wirkstoffdosis zwischen etwa 0,5 und 10 mg/kg Körpergewicht bei oraler Gabe und zwischen etwa 0,01 und 1,0 mg/kg Körpergewicht bei parenteraler Gabe. Im Normalfall werden mit täglichen Dosen von 1 bis 5 mg/kg oral und 0,02 bis 0,1 mg/kg parenteral zufriedenstellende Ergebnisse erzielt.

Die neuen Verbindungen können in den gebräuchlichen galenischen Applikationsformen fest oder flüssig angewendet werden, z.B. als Tabletten, Filmtabletten, Kapseln, Pulver, Granulate, Dragees, Suppositorien oder Lösungen. Diese werden in üblicher Weise hergestellt. Die Wirkstoffe können dabei mit den gebräuchlichen galenischen Hilfsmitteln wie Tablettenbindern, Füllstoffen, Konservierungsmitteln, Tablettensprengmitteln, Fliessregulierungsmitteln, Weichmachern, Netzmitteln, Dispergiermitteln, Emulgatoren, Lösungsmitteln, Retardierungsmitteln und/oder Antioxidantien verarbeitet werden (vgl. L.G. Goodman, A. Gilman: The Pharmacological Basis of Therapeutics). Die so erhaltenen Darreichungsformen enthalten den Wirkstoff normalerweise in einer Menge von 0,1 bis 99 Gew.-%.

Die errfindungsgemässe Verbindung und ihre physiologisch verträglichen Säureadditionssalze sind stark antiarrythmisch wirksam und eignen sich daher insbesondere zur Pharmakotherapie von Herzrhythmusstörungen.

Die antiarrhythmische Wirksamkeit der erfindungsgemässen Verbindung wird an isolierten linken Vorhöfen von männlichen Meerschweinchen (Stamm: Pirbright white, Gewicht 350 bis 450 g) bestimmt. In einem Organbad (Volumen 125 ml) mit Carbogen-gesättigter Tyrode-Lösung (pH 7,4, Temperatur 32°C) suspendierte Vorhöfe sind mit einem Gewicht von 1,0 g vorbelastet und durch Rechteckimpulse von 1 Hz Grundrhythmus und doppelten Reizschwellenwerten (Rheobase: 0,2 bis 1,4 V, Chronaxie: 0,3 bis 0,5 msec) angetrieben. Als Kriterium für antiarrhythmische Wirksamkeit wird durch automatische kontinuierliche Frequenzerhöhung die Frequenz (Hz) bestimmt, bis zu der auf jeden Reiz eine Kontrakton folgt (maximale Folgefrequenz, MFF). Aus der linearen Beziehung zwischen log Konzentration (mg/l) der Wirksubstanz und der relativen Abnahme der maximalen Folgefrequenz (Δ%) wird die Konzentration berechnet, welche eine Abnahme der MFF um 50% bewirkt (EC 50%).

Ferner wird aus der linearen Beziehung zwischen log Konzentration (mg/l) und der relativen Änderung der Kontraktionsamplitude (Δ%) als Mass für negativ inotrope Wirkung die Konzentration berechnet, welche eine Abnahme der Kontraktionsamplitude um 25% bewirkt (EC 25%).

Darüber hinaus wird die antiarrhythmische Wirksamkeit der neuen Substanz an männlichen Ratten (Stamm: Sprague Dawley, Gewicht 200

bis 230 g) nach oraler Gabe bestimmt. 45 min nach Substanzapplikation werden die Tiere mit Thiobutabarbital-Natrium (100 mg/kg i.p.) narkotisiert. Als arrhythmogene Substanz dient Aconitin, das 60 min nach Substanzapplikation i.v. fundiert wird (Dosierungsgeschwindigkeit: 0,005 mg/kg × min). Bei unbehandelten Tieren (N = 52) treten nach 2,74±0,07 min im EKG Arrhythmien auf, deren Eintritt durch Antiarrhythmika dosisabhängig verzögert werden kann. Aus der linearen Beziehung zwischen log Dosis (mg/kg) der Prüfsubstanz und der relativen Verlängerung der Aconitin-Infusionsdauer (%) wird die Dosis bestimmt, welche die Infusionsdauer um 50% verlängert (ED 50%).

Ferner wird die Dosis ermittelt, bei welcher toxische Symptome (Veränderungen im Ausgangs-EKG, Cyanose, Krämpfe) auftreten. Als Mass für die Verträglichkeit wird daher der Quotient aus akuter toxischer Dosis und antiarrhythmischer ED 50% bestimmt.

Zur weiteren Charakterisierung der neuen Verbindung wird untersucht, ob neben antiarrhythmischen Eigenschaften erregende Effekte an glatter Muskulatur, die zu unerwünschten Nebenwirkungen (Blutdrucksteigerung, Uterusstimulation) führen können, nachweisbar sind. An in einem Organbad mit Carbogen-gesättigter Krebs-Henseleit-Lösung (37°C) suspendierten Spiralstreifen der Rattenaorta wird die Konzentration (mol/l) bestimmt, die 50% der Kontraktion einer zuvor getesteten Noradrenalin-Konzentration ($10^{-6}$ mol/l) erreicht (EC 50%).

Ausserdem wird die Wirkung der erfindungsgemässen Verbindung auf den systolischen und diastolischen Blutdruck von wachen Hunden bei i.v.-Applikation geprüft.

Als Vergleichssubstanzen dienten die bekannten antiarrhythmisch wirksamen Verbindungen N,N'-Bisbenzylbispidin (B, DE-OS 2 428 792), N-Benzyl-N'-(2,2-diphenylethyl)-bispidin (A, DE-OS 2 726 571) und Spartein.

Am isolierten Meerschweinchenvorhof (Tab. 1) wirkt das N-(4-Aminobenzoyl)-N'-benzylbispidin (C) stark antiarrhythmisch und bewirkt in Analogie zu den Referenzsubstanzen eine Abnahme der MFF. Besonders bemerkenswert ist die relativ geringe Abnahme der Kontraktionskraft durch diese Substanz. Dadurch ist der Abstand zwischen erwünschter antiarrhythmischer und unerwünschter negativ inotroper Wirkung – ersichtlich aus dem Quotienten EC 25% (Abnahme der Kontraktionskraft)/EC 50% (Abnahme der MFF) – für die erfindungsgemässe Verbindung 4- bzw. 5mal grösser als bei den Vergleichsverbindungen.

An der Aconitinarrhythmie der Ratte (Tab. 2) übertrifft die antiarrhythmische Wirkung der erfindungsgemässen Verbindung die der Referenzsubstanz B etwa um das 4fache, der Referenzsubstanz A um dasDoppelte und die des Sparteins um das 18fache an Wirksamkeit bei etwa doppeltem bzw. gleichem Abstand zwischen toxischer und antiarrhythmisch wirksamer Dosis.

Ein weiterer Vorteil der neuen Verbindung ist das Fehlen erregender Effekte an der glatten Muskulatur (Tab. 3). Im Unterschied zu Referenzsubstanz B und Spartein hat diese Verbindung selbst in sehr hoher Konzentration ($3 \cdot 10^{-4}$ mol/l) keine konstriktorische Wirkung an arterieller Gefässmuskulatur in vitro. Dieser Unterschied gilt offensichtlich auch für den intakten Organismus. Im Gegensatz zu Referenzsubstanz B und Spartein (Tab. 4), welche zu einer deutlichen Steigerung des systolischen und insbesondere des diastolischen Blutdrucks führen, hat die erfindungsgemässe Verbindung keine blutdrucksteigernde Wirkung. Hieraus folgt eine bessere Verträglichkeit bei der Therapie von Rhythmusstörungen.

Tabelle 1
Wirkung am isolierten Meerschweinchenvorhof

| Substanz | MFF | | Kontraktionskraft | | Q[d)] |
|---|---|---|---|---|---|
| | EC 50%[a)] | R.W.[b)] | EC 25%[c)] | R.W. | |
| C | 6,14 | 0,96 | 100 | 0,22 | 16,3 |
| A | 1,43 | 3,93 | 4,92 | 4,43 | 3,44 |
| B | 5,92 | 1,00 | 21,8 | 1,00 | 3,68 |

a) Konzentration (mg/l), welche eine 50%ige Abnahme der maximalen Folgefrequenz bewirkt
b) R.W. = Reaktive Wirksamkeit: Referenzsubstanz B = 100
c) Konzentration (mg/l), welche eine Abnahme der Kontraktionskraft um 25% bewirkt
d) $Q = \frac{EC\ 25\%}{EC\ 50\%}$

Tabelle 2
Antiarrhythmische Wirkung und Toxizität an der Ratte
Appl.: Per os

| Substanz | Wirksame Dosis | | Toxische Dosis | Q[d)] |
|---|---|---|---|---|
| | EC 50%[a)] | R.W.[b)] | | |
| C | 6,37 | 3,56 | 46,4 | 7,3 |
| A | 13,0 | 1,75 | 100 | 7,7 |
| B | 22,7 | =1,00 | 100 | 4,4 |
| Spartein | 112 | 4,93 | 464 | 4,1 |

a) Dosis (mg/kg) welche die Aconitin-Infusionsdauer um 50% verlängert
b) R.W. = Relative Wirksamkeit: Referenzsubstanz B = 1,00
c) Dosis (mg/kg) nach deren Applikation die ersten toxischen Symptome beobachtet werden
d) $Q = \frac{\text{Toxische Dosis}}{\text{ED 50\%}}$

Tabelle 3
Konstriktorische Wirkung am isolierten Aortenstreifen der Ratte

| Substanz | EC 50 mol/1 |
|---|---|
| C | –a) |
| Spartein | $5{,}5.10^{-4}$ |
| B | $3{,}8.10^{-6}$ |

a) bis $3.10^{-4}$ mol/l kein Effekt

Tabelle 4
Blutdruckwirkung am wachen Hund
Appl.: i.v.

| Substanz | mg/kg | Blutdruckänderung (Δ mmHg) | |
|---|---|---|---|
| | | Syst. | Diast. |
| C | 2,0 | – 5 | – 2 |
| B | 2,0 | +20 | +59 |
| Spartein | 5,0 | +19 | +40 |

Die folgenden Beispiele erläutern die Erfindung.

Beispiel 1

a) N-(4-Nitrobenzoyl)-N'-benzylbispidin

Zu einer Lösung von 84,7 g (0,392 Mol) Monobenzylbispidin in 800 ml Dimethylformamid wurden 18,8 g (0,43 Mol) einer 55%igen Natriumhydridsuspension gegeben. Nach fünfstündigem Rühren bei Raumtemperatur wurde eine Lösung von 72,7 g (0,392 Mol) 4-Nitrobenzoylchlorid in 200 ml Dimethylformamid zugetropft und weitere drei Stunden gerührt. Das überschüssige Natriumhydrid wurde mit Methanol zersetzt. Nach dem Abdestillieren der Lösungsmittel im Vakuum wurde der Rückstand in Wasser aufgenommen und sofort mit Ether extrahiert. Nach dem Trocknen mit Natriumsulfat wurde der Ether verdampft. Als Rückstand wurden 118,0 g (82,5%) N-(4-Nitrobenzoyl)-N'-benzylbispidin erhalten. Fp. 125 bis 127°C.

b) N-(4-Aminobenzoyl)-N'-bnezylbispidin-1,5-fumarat

118,0 g N-(4-Nitrobenzoyl)-N'-benzylbispidin wurden in 1700 ml Ethanol gelöst und nach Zugabe von 5 g 5% Pt/C in einem 2 l-Kolben unter Rühren hydriert. Nach beendeter Wasserstoffaufnahme wurde vom Katalysator abfiltriert und eingedampft. Der Rückstand wurde in 1000 ml heissem Isopropanol gelöst und mit 56,2 g Fumarsäure versetzt. Beim Abkühlen der Lösung kristallisierten 124,0 g (81%) N-(4-Aminobenzoyl)-N'-benzylbispidin-1,5-fumarat aus. Fp. 151–153°C.

Beispiel 2

Auf einer Tablettenpresse werden in üblicher Weise Tabletten folgender Zusammensetzung gepresst:

50 mg N-(4-Aminobenzoyl)-N'-benzylbispidin
120 mg Maisstärke
13,50 mg Gelatine
45 mg Milchzucker
22,5 mg Talk
2,25 mg Aerosil® (chemisch reine Kieselsäure in submikroskopisch feiner Verteilung)
6,75 mg Kartoffelsätrke (als 6%iger Kleister)

Beispiel 3

In üblicher Weise werden Dragees folgender Zusammensetzung hergestellt:

30 mg N-Benzoyl-N'-benzylbispidin
60 mg Kernmasse
60 mg Verzuckerungsmasse

Die Kernmasse besteht aus 9 Teilen Maisstärke, 3 Teilen Milchzucker und 1 Teil Luviskol® VA 64 (Vinylpyrrolidon-Vinylacetat-Mischpolymerisat 60:40, vgl. Pharm. Ind. 1962, 586). Die Verzuckerungsmasse besteht aus 5 Teilen Rohrzucker, 2 Teilen Maisstärke, 2 Teilen Calciumcarbonat und 1 Teil Talk. Die so hergestellten Dragees werden anschliesend mit einem magensaftresistenten Überzug versehen.

Beispiel 4

10 g N-(4-Aminobenzoyl)-N′-benzylbispidin werden in 5000 ml Wasser unter Zusatz von NaCl und einer äquimolaren Menge Essigsäure gelöst, so dass eine blutisotonische Lösung entsteht. Jeweils 5 ml der Lösung werden in Ampullen gefüllt und sterilisiert.

**Patentansprüche**

1. N-(4-Aminobenzoyl)-N′-benzylbispidin der Formel I

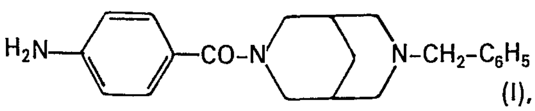

(I),

sowie dessen Salze mit physiologisch verträglichen Säuren.

2. Verfahren zur Herstellung von N-(4-Aminobenzoyl)-N′-benzylbispidin der Formel I und dessen Salzen mit physiologisch verträglichen Säuren, dadurch gekennzeichnet, dass man N-Monobenzylbispidin mit einer Verbindung der Formel II

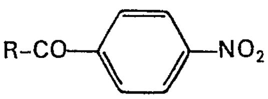

(II),

worin R ein Halogenatom bedeutet, umsetzt, die Nitrogruppe reduziert und die so erhaltene Verbindung gegebenenfalls in ihre Salze mit physiologisch verträglichen Säuren überführt.

3. Arzneimittel, enthaltend eine Verbindung der Formel I gemäss Anspruch 1.

4. Verbindung der Formel I gemäss Anspruch 1 zur Verwendung bei der Heilung von Herzkrankheiten.

**Revendications**

1. N-(4-aminobenzoyl)-N′-benzylbispidine de formule I

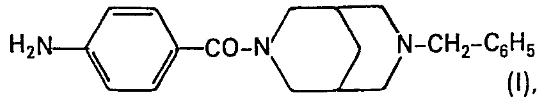

(I),

ainsi que ses sels d'acides compatibles physiologiquement.

2. Procédé de préparation de N-(4-aminobenzoyl)-N′-benzylbispidine de formule I et ses sels d'acides compatibles physiologiquement, caractérisé par le fait qu'on fait réagir de la N-monobenzylbispidine avec un composé de formule II

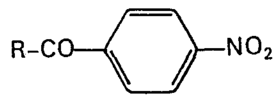

(II),

où R représente un atome d'halogène, on réduit le groupe nitro et l'on transforme éventuellement le composé ainsi obtenu en ses sels d'acides compatibles physiologiquement.

3. Médicament, contenant un composé de formule I selon la revendication 1.

4. Composé de formule I selon la revendication 1 pour utilisation dans le traitement des maladies du cœur.

**Claims**

1. N-(4-Aminobenzoyl)-N′-benzylbispidine of the formula I

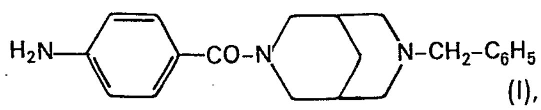

(I),

and its salts with physiologically tolerated acids.

2. A process for the preparation of N-(4-aminobenzoyl)-N′-benzylbispidine of the formula I and its salts with physiologically tolerated acids, wherein N-monobenzylbispidine is reacted with a compound of the formula II

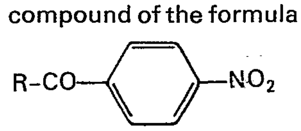

(II),

where R is halogen, the nitro is reduced, and the compound thus obtained is converted, if required, into its salt with a physiologically tolerated acid.

3. A drug containing a compound of the formula I as claimed in claim 1.

4. A compound of the formula I as claimed in claim 1 for use in curing cardiac disorders.